**Europäisches Patentamt**

**European Patent Office**

**Office européen des brevets**

(11) Veröffentlichungsnummer: **0 302 958 B1**

# EUROPÄISCHE PATENTSCHRIFT

(45) Veröffentlichungstag der Patentschrift: **20.05.92**

(51) Int. Cl.⁵: **A61M 15/00**

(21) Anmeldenummer: **87111686.9**

(22) Anmeldetag: **12.08.87**

---

(54) **Transportables Inhalationsgerät.**

---

(43) Veröffentlichungstag der Anmeldung:
**15.02.89 Patentblatt 89/07**

(45) Bekanntmachung des Hinweises auf die
Patenterteilung:
**20.05.92 Patentblatt 92/21**

(84) Benannte Vertragsstaaten:
**AT BE CH DE FR GB LI NL SE**

(56) Entgegenhaltungen:
DE-A- 2 146 256    FR-A- 2 554 351
US-A- 3 301 255    US-A- 3 705 689
US-A- 4 197 842    US-A- 4 396 015

(73) Patentinhaber: **Brugger, Stephan,
Dipl.-Wirt.-Ing.
Etztalstrasse 21
W-8137 Berg(DE)**

(72) Erfinder: **Der Erfinder hat auf seine Nennung
verzichtet**

(74) Vertreter: **Hoffmann, Klaus, Dr. rer. nat. et al
Hoffmann . Eitle & Partner Patentanwälte
Postfach 81 04 20 Arabellastrasse 4
W-8000 München 81(DE)**

---

Rank Xerox (UK) Business Services
(3.08/2.19/2.0)

**Beschreibung**

Die Erfindung betrifft ein transportables Inhalationsgerät zur Behandlung erkrankter Atemwege, mit einem Gehäuse, einem Drucklufterzeuger, einem Elektromotor, der den Drucklufterzeuger antreibt, einer Stromversorgung mit wenigstens einem Akkumulator, einem vom Gehäuse getrennten Aerosol-Vernebler, und mit einem flexiblen Druckluftschlauch, dessen eines Ende mit dem Drucklufterzeuger in Verbindung steht und dessen anderes Ende an den Aerosol-Vernebler angeschlossen ist.

Ein derartiges tragbares Inhalationsgerät ist bereits aus der US-PS 4 257 415 bekannt. Der eingebaute Akkumulator erlaubt Inhalationsbehandlungen unabhängig vom Netzstrom, beispielsweise auf Reisen, im Auto oder im Boot. Vor Beginn der Behandlung muß der Aerosol-Vernebler mit Medikantenlösung gefüllt werden; mittels der vom Drucklufterzeuger bereitgestellten Preßluft wird diese Medikamentenlösung zu einem feinen Aerosol-Nebel zerstäubt, den der Patient zwecks Behandlung der Atemwege inhaliert. Bei dem bekannten Gerät wird der Drucklufterzeuger bzw. der diesen antreibende Elektromotor mittels eines außen am Gehäuse angeordneten Schalters ein- bzw. ausgeschaltet.

Die Erzeugung von Druckluft erfordert relativ viel Energie, welche dem antreibenden Elektromotor zugeführt werden muß. Speziell bei tragbaren netzunabhängigen Inhalationsgeräten kommt es deshalb darauf an, mit der im Akkumulator gespeicherten elektrischen Energie möglichst sparsam umzugehen. Eine wirksame atemtherapeutische Behandlung durch Inhalieren von medikamentenhaltigen Aerosol dauert nämlich ungefähr 15 min. Ein ununterbrochener Betrieb des Drucklufterzeugers über diese Zeitspanne würde dazu führen, daß nur sehr wenige Inhalationsbehandlungen durchgeführt werden könnten, bis die Kapazität des eingebauten Akkumulators erschöpft wäre. Man wird deshalb bestrebt sein, den Elektromotor des Drucklufterzeugers nur dann laufen zu lassen, wenn es zur Aerosol-Erzeugung unbedingt notwendig ist, also während des Einatmens; während des darauffolgenden Ausatemvorgangs kann die Erzeugung von Druckluft unterbrochen werden. Ein fortwährendes Betätigen eines Ein- und Ausschalters, wie er am Gehäuse des vorbekannten Inhalationsgeräts selbst angeordnet ist, ist unpraktisch. Der Patient wird stattdessen aus Bequemlichkeit dazu neigen, während des Ausatmens lediglich den Aerosol-Vernebler vom Munde abzusetzen und das Gerät einfach weiterlaufen zu lassen, so daß ungefähr die Hälfte der Zeit Aerosol nutzlos in die Umgebungsluft geblasen wird. Die Folge ist nicht nur eine Vergeudung wertvoller Medikamente und unerwünschte Kontamination der Umgebungsluft, sondern auch eine vorzeitige Erschöpfung der Batteriereserven.

Nun ist es aus US-A-3 303 255 auch schon bekannt, bei einem transportablen Inhalationsgerät ähnlicher Bauart für den Betrieb des Drucklufterzeugers einen Elektromotor vorzusehen, der mit Netzstrom betrieben wird. Bei diesem Gerät entfällt zwar das Problem einer vorzeitigen Erschöpfung der Batteriereserven, jedoch kann es nur bei Vorhandensein einer Netzstromsteckdose eingesetzt werden. Es erfordert darüber hinaus hinreichenden Schutz des Patienten vor Stromschlag bei beschädigter elektrischer Isolation oder unsachgemäßem Anschluß. Im übrigen stellen sich auch dort die beschriebenen Probleme im Zusammenhang mit der Handbetätigung eines Ventils zum Sperren der Druckluftzufuhr.

Schließlich ist aus FR-A-2 554 351 ein Inhalationsgerät bekannt, bei dem Druckfühler vorgesehen sind, um das Druckniveau für die Ein- und Ausatemphasen zwischen einem Maximum und einem Minimum zu regeln.

Der vorliegenden Erfindung liegt die Aufgabe zugrunde, ein transportables Inhalationsgerät der eingangs genannten Art zu schaffen, das bei vorgegebener Kapazität des eingebauten Akkumulators eine möglichst lange Behandlungsdauer erlaubt und sich durch komfortable Bedienbarkeit auszeichnet.

Erfindungsgemäß wird diese Aufgabe gemäß dem kennzeichnenden Teil von Patentanspruch 1 dadurch gelöst, daß bei einem Inhalationsgerät der eingangs genannten Art folgende weitere Merkmale vorgesehen werden: Ein am Aerosol-Vernebler angeordnetes, handbetätigtes Ventil, das die Zufuhr von Druckluft absperrt, eine elektronische Steuereinheit zur Steuerung des Elektromotors, einen zwischen Drucklufterzeuger und Ventil angeordneten Sensor, der den Druck im Druckluftschlauch überwacht und der elektrisch mit der Steuereinheit verbunden ist, und einen in der Steuereinheit enthaltenen Regler, der bei Druckabfall im Druckluftschlauch den Elektromotor des Drucklufterzeugers anschaltet und bei Erreichen eines oberen Grenzwertes im Druckluftschlauch den Elektromotor abschaltet.

Zu Beginn der Inhalationsbehandlung wird der Aerosol-Vernebler mittels des flexiblen Druckluftschlauchs an den im Gehäuse befindlichen Drucklufterzeuger angeschlossen und das Gerät eingeschaltet. Der hinter dem Drucklufterzeuger angeordnete Drucksensor meldet einen zu geringen Druck innerhalb des Schlauchs, so daß die Steuereinheit automatisch den Elektromotor anlaufen läßt, bis der Drucklufterzeuger mindestens den zur Aerosol-Vernebelung benötigten Betriebsdruck aufgebaut hat. Das am Aerosol-Vernebler angeordnete Ventil ist hierbei geschlossen, so daß zunächst keine Druckluft entweichen kann. Gesteuert vom Drucksensor und der nachgeschalteten Steuereinheit schaltet der Elektromotor nach kürzester Zeit wieder ab. Das Gerät ist betriebsbereit. - Zur Inhalationsbehandlung selbst nimmt der Patient den Aerosol-Vernebler in die Hand und führt dessen Ausblasöffnung zum Munde. Bei Betätigung des griffgünstig plazierten Ventils strömt sofort die bereitstehende Druckluft in den Aerosol-Vernebler ein, in welchem sie mit der Medikamentenlösung zu dem gewünschten Aerosol zerstäubt wird. Der infolge des Entweichens der Druckluft in den Aerosol-Venebler rasch eintretende Druckabfall innerhalb des Druckluftschlauchs wird vom Drucksensor erfaßt, worauf die Steuereinheit den Elektromotor des Drucklufterzeugers einschaltet, damit der Druckabfall möglichst rasch ausgeglichen wird. Solange der Patient inhaliert, bleibt das Ventil am Aerosol-Vernebler geöffnet, so daß ständig Druckluft vom Drucklufterzeuger über den Druckluftschlauch nachströmt. Atmet der Patient anschließend in die freie Umgebung aus, so wird die Zufuhr von Druckluft in den Aerosol-Vernebler mittels des handbetätigten Ventils abgesperrt. Die Folge ist eine nahezu schlagartige Erhöhung des Drucks innerhalb des Druckluftschlauchs. Bei Erreichen des vorgegebenen Maximaldrucks wird der Elektromotor im Gerät sofort abgeschaltet, und zwar so lange, bis der Drucksensor erneut einen Druckabfall infolge Öffnen des Ventils am Aerosol-Vernebler registriert.

Das automatische An- bzw. Abschalten des Elektromotors im Rhythmus des Ein- bzw. Ausatmens, ausgelöst durch die Betätigung des Ventils am Aerosol-Vernebler, hat den Vorteil, daß der Drucklufterzeuger nur dann Luft verdichtet, wenn diese tatsächlich zur Aerosol-Erzeugung benötigt wird. Die Erzeugung von Druckluft kann vom Patienten jederzeit auf einfache und bequeme Art durch Betätigung des Ventils am Aerosol-Vernebler unterbrochen werden. Eine griffgünstige Aufstellung des Inhalationsgeräts selbst ist deshalb nicht mehr notwendig. Bei einem üblichen Zeitverhältnis von Inspiration zu Exspiration von ungefähr 1:1 lassen sich mit dem erfindungsgemäß ausgestalteten Inhalationsgerät infolge des intermittierenden Antriebs des Drucklufterzeugers mindestens doppelt so viel Inhalationen durchführen, als dies mit einem vergleichbaren Gerät ohne drucküberwachte Steuerung des elektromotorischen Antriebs möglich wäre.

Bei einer bevorzugten Ausführungsform der Erfindung ist der Regler insgesamt als Zweipunkt-Regler ausgebildet, so daß bei Druckabfall im Druckluftschlauch unter einen unteren Grenzwert der Elektromotor des Drucklufterzeugers angeschaltet und bei Erreichen eines oberen Grenzwertes für den Druck wieder abgeschaltet wird. Eine derartige Druckregelung zwischen einem unteren und einem oberen Grenzwert läßt sich schaltungstechnisch einfach realisieren und arbeitet in der Praxis zuverlässig.

Es hat sich als zweckmäßig erwiesen, wenn der Elektromotor bei einem Abfall des Drucks innerhalb des Druckschlauchs unter 0,7 bar eingeschaltet und bei einem Druck von mehr als 1 bar wieder abschaltet. Da bei geschlossenem Ventil am Aerosol-Vernebler somit stets ein Druck von mindesters 1 bar ansteht, setzt die Aerosol-Vernebelung ohne Zeitverzug in unmittelbarem Ansprechen auf die Betätigung des Ventils ein. Die gewählte Druckdifferenz von 0,3 bar zwischen oberem und unterem Grenzwert für den Druck verhindert ein übermäßig empfindliches Ansprechverhalten der Steuereinheit auf Druckschwankungen.

Als Elektromotor findet vorzugsweise ein Gleichstrommotor Verwendung. Als Drucklufterzeuger gelangen in der Regel speziell für derartige Inhalationsgeräte konstruierte Membrankompressoren zum Einsatz.

Bei einer weiter bevorzugten Ausführungsform der Erfindung ist der Drucksensor innerhalb des Gehäuses angeordnet. Alle elektrischen Bauelemente und die zugehörigen Anschlüsse und Verbindungsleitungen, welche in der Regel anfällig gegen Feuchtigkeit und Verschmutzung sind, befinden sich so innerhalb des schützenden Gehäuses. Die mit Medikamentenlösung oder Aerosol in Berührung kommenden Teile wie Aerosol-Vernebler, Ventil, Druckschlauch funktionieren rein mechanisch. Dank dieser konsequenten Trennung von elektrischen und mechanischen Bauelementen zeichnet sich das erfindungsgemäße Inhalationsgerät durch Robustheit und besonders geringe Störanfälligkeit aus.

In vorteilhafter Ausführung des erfindungsgemäßen Inhalationsgeräts ist der Drucksensor ein Piezo-Element, dessen Ausgangsspannung unmittelbar von einer elektronischen Steuereinheit für den Elektromotor verarbeitet werden kann.

Der Bedienungskomfort wird weiter erhöht, wenn das Ventil am Aerosol-Vernebler durch Druck auf eine Drucktaste betätigt wird. Bevorzugt wird eine Ausführung, bei dem das Ventil bei gedrückter Drucktaste öffnet, so daß Druckluft vom Druckluftschlauch in den Aerosol-Vernebler einströmt, und beim Loslassen der Drucktaste automatisch wieder schließt. So wird gewährleistet, daß beim Weglegen des Aerosol-Verneblers das Gerät immer sofort automatisch abschaltet.

Bei einer weiteren Ausführungsform der Erfindung ist der Druckluftschlauch über eine an der Außenseite des Gehäuses angeordnete Steckbuchse mit dem Drucklufterzeuger verbunden. Zu Reinigungszwecken und zum Transport läßt sich der Druckluftschlauch mit dem daran angeschlossenen Aerosol-Vernebler schnell und einfach vom übrigen Gerät trennen.

Trotz der erfindungsgemäßen Auslegung der Antriebssteuerung des Druckerzeugers wird der eingebaute Akkumulator nach der Durchführung von einigen Inhalationsbehandlungen erschöpft sein. Bei einer besonders praktischen Weiterbildung der Erfindung umfaßt deshalb die eingebaute Stromversorgung ein Ladegerät für den Akkumulator und ein Netzteil. Die Betriebsbereitschaft kann dann durch Aufladen an der nächsten Netzsteckdose wieder hergestellt werden, ohne daß ein separates Ladegerät mitgeführt werden müßte. Als zusätzlicher Vorteil ergibt sich die Möglichkeit, das Inhalationsgerät trotz leerem Akkumulator auch direkt aus dem Stromnetz speisen zu können.

Anstelle des bei herkömmlichen Geräten vorhandenen Schalters zum direkten Ein- bzw. Ausschalten des elektromotorischen Antriebs des Druckerzeugers ist bei dem erfindungsgemäßen Inhalationsgerät ein Einschalter zur Aktivierung der elektronischen Steuerung vorgesehen, welcher sich vorzugsweise an der Außenseite des Gehäuses befindet. Die hierdurch mögliche Unterbrechung der Spannungsversorgung verhindert unabsichtlichen Leerlauf des Geräts, beispielsweise bei versehentlich nicht druckdicht angeschlossenem Aerosol-Vernebler oder beschädigtem Druckluftschlauch.

Ein Ausführungsbeispiel der Erfindung wird nachstehend anhand der beigefügten Zeichnungen näher erläutert. Es zeigen:

Fig. 1 Ein transportables Inhalationsgerät, in einer perspektivischen Darstellung;

Fig. 2 ein vereinfachtes Blockschaltbild des Inhalationsgeräts von Fig. 1.

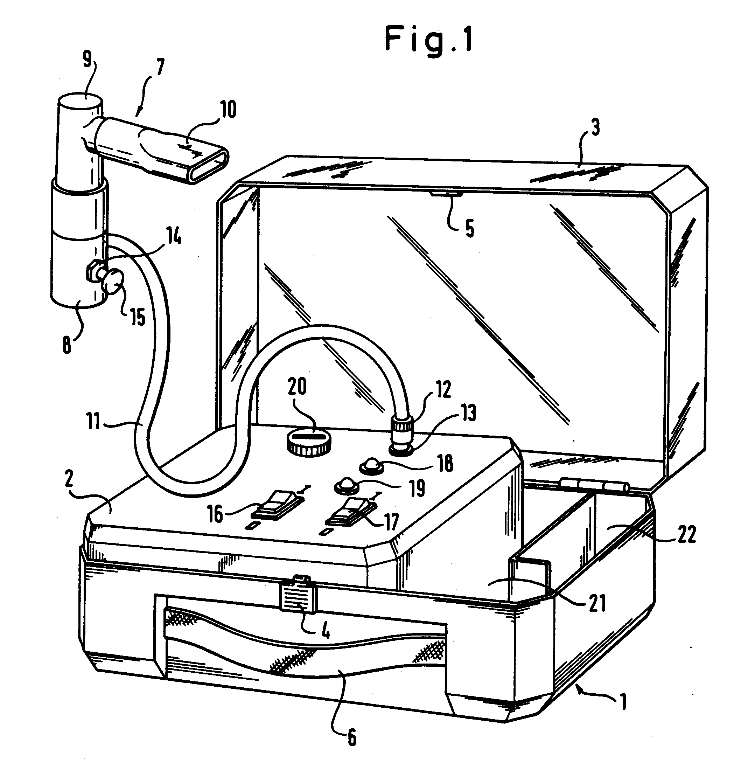

Das in Fig. 1 dargestellte transportable Inhalationsgerät besitzt ein Gehäuse 1 aus Kunststoff, in dem alle elektrischen Bauelemente untergebracht sind. An seiner Oberseite weist das Gehäuse 1 eine ebene Deckplatte 2 auf. An der Rückseite des Gehäuses 1 ist ein Klappdeckel 3 aus durchsichtigem Kunststoff schwenkbeweglich angelenkt, der in geschlossenem Zustand die Deckplatte 2 überdeckt. Ein Schnappverschluß 4 verhindert im Zusammenwirken mit einer kleinen Nase 5 ein Aufspringen des geschlossenen Klappdeckels 3. Vorne am Gehäuse 1 ist ein Tragegriff 6 befestigt.

Zum Inhalationsgerät gehört ein Aerosol-Vernebler 7, der vom Patienten in die Hand genommen wird. Dieser besteht aus einem Unterteil 8, welches die zu vernebelnde Medikamenten-Lösung enthält, einem Oberteil 9 sowie einem Mundstück 10. Der Zufuhr von Druckluft in das Innere des Aerosol-Verneblers 7 dient ein Druckluftschlauch 11. Das freie Ende des Druckluftschlauchs 11 mündet in eine Kupplung 12, welche in eine auf der Deckplatte 2 des Gehäuses 1 angeordnete Steckbuchse 13 eingesteckt ist. Im Unterteil 8 des Aerosol-Verneblers 7 ist ferner ein Ventil 14 vorgesehen, das die Zufuhr von Druckluft in das Innere des Aerosol-Verneblers 7 absperrt. Bei Druck auf die unter Vorspannung einer Feder stehenden Drucktaste 15 öffnet das Ventil 14, um beim Loslassen sofort wieder automatisch abzusperren.

Auf der Deckplatte 2 des Gehäuses 1 ist ein Netzschalter 16, ein Einschalter 17 sowie eine Akkumulator-Kontrolleuchte 18 und eine Lade-Kontrolleuchte 19 angeordnet. Mit 20 ist der Schraubverschluß eines auswechselbaren Luftfilters bezeichnet. Ein nach oben hin offenes Fach 21 dient zur Aufnahme des Aerosol-Verneblers 7 während des Transports des Inhalationsgeräts. Daneben ist ein weiteres Fach 22 für Zubehör vorgesehen.

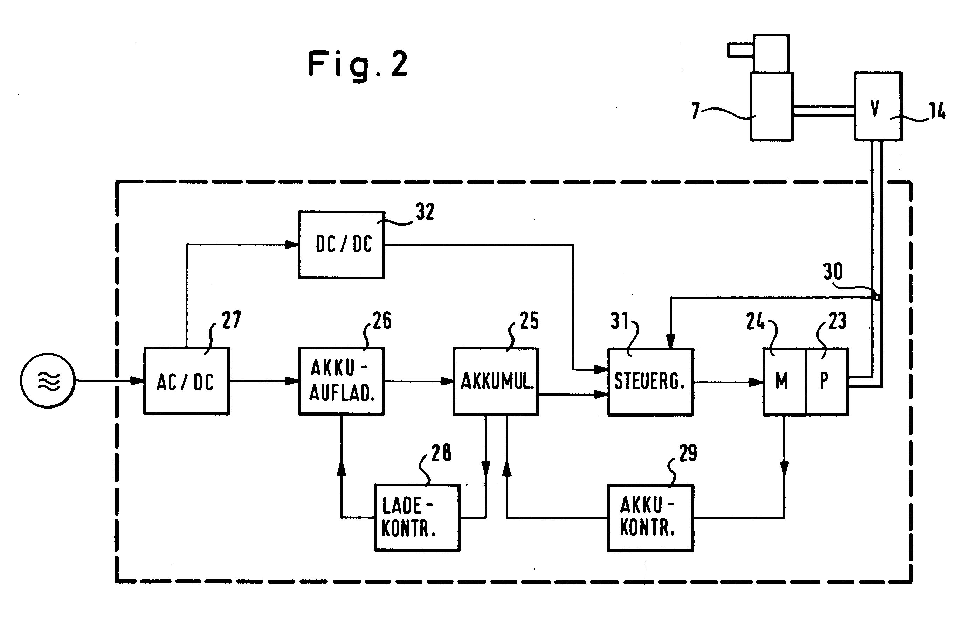

Im Blockschaltbild von Fig. 2 sind die elektrischen und mechanischen Komponenten des Inhalationsgeräts schematisch dargestellt. Kernstück ist ein Druckerzeuger 23, der beispielsweise als Membrankompressor ausgeführt ist. Als Antriebsaggregat dient ein angeflanschter Elektromotor 24 für Gleichspannung. Die Stromversorgung umfaßt einen wiederaufladbaren Akkumulator 25, ein Ladegerät 26 sowie ein Netzteil 27 zur Umwandlung der Netz-Wechselspannung in die benötigte Gleichspannung. Eine Ladekontrolle 28 überwacht die Aufladung des Akkumulators 25. Der Ladevorgang wird durch Aufleuchten der Lade-Kontrolleuchte 19 auf der Deckplatte 2 des Gehäuses 1 angezeigt. Der Überwachung des Ladezustands des Akkumulators 25 dient eine Akkumulator-Kontrolle 29, welche bei Unterschreiten einer bestimmten Mindestspannung die Akkumulator-Kontrolleuchte 18 aufleuchten läßt (vgl. Fig. 1).

Die Druckkammer des Druckerzeugers 23 ist über den aufgesteckten Druckluftschlauch 11 mit dem Aerosol-Vernebler 7 verbunden, wobei das Ventil 14 die Druckluftzufuhr absperrt. Zwischen dem Druckerzeuger 23 und dem handbetätigten Ventil 14 ist ein Drucksensor 30 angeordnet, der als Piezo-Element ausgebildet ist. Elektrisch ist dieser Drucksensor 30 mit einer elektronischen Steuereinheit 31 verbunden, welche den Elektro-Motor 24 ansteuert. Bei netzunabhängigem Betrieb erhält die elektronische Steuereinheit 31 den Betriebsstrom für den Elektromotor 24 vom Akkumulator 25 zugeführt. Ist Netzstrom verfügbar, so erfolgt die Stromversorgung über das Netzteil 27 und einen zwischengeschalteten Gleichspannungswandler 32.

Nach Einschalten des Einschalters 17 treibt der Elektromotor 24 - gesteuert von der elektronischen Steuereinheit 31 - den Druckerzeuger 23 so lange an, bis in der am Ende des durch das Ventil 14 dicht abgeschlossenen Druckluftschlauchs 11 ein Innendruck von ungefähr 1 bar herrscht. Im Ansprechen auf den Drucksensor 30 schaltet die Steuereinheit 31, die einen Zweipunkt-Regler enthält, den Elektromotor 24 ab. Öffnet der Patient nun das Ventil 14 durch Betätigung dessen Drucktaste 15 (vgl. Fig. 1), strömt Druckluft aus dem Druckluftschlauch 11 in den Aerosol-Vernebler 7. Die Aerosol-Vernebelung setzt augenblicklich ein; der erzeugte Medikamenten-Nebel kann vom Patienten durch das Mundstück 10 eingeatmet werden. Infolge des Entweichens von Druckluft in den Aerosol-Vernebler 7 fällt der Druck im Druckluftschlauch 11 ab. Bei Erreichen des vorgesehenen Mindestdrucks von ungefähr 0,7 bar schaltet die Steuereinheit 31 im Ansprechen auf den Drucksensor 30 den Elektromotor 24 wieder ein, so daß der Drucklufterzeuger 23 erneut Preßluft in den Druckluftschlauch 11 pumpt. Auf diese Weise wird der zur Aerosol-Vernebelung benötigte Betriebsdruck aufrechterhalten.

Läßt der Patient die Drucktaste 15 des Ventils 14 los, wird die Druckluftzufuhr - und damit die Aerosol-Erzeugung - augenblicklich unterbrochen. In dem nunmehr wieder druckdicht abgeschlossenen Druckluftschlauch 11 steigt der Druck nahezu schlagartig an. Bei Erreichen des eingestellten Maximaldrucks von ungefähr 1 bar, schaltet die Steuereinheit 31 den Elektromotor 24 ab. Der Drucklufterzeuger 23 wird erst dann wieder angetrieben, wenn der Patient erneut inhalieren will und hierzu das Ventil 14 durch erneutes Drücken dessen Drucktaste 15 wieder öffnet. Solange das Inhalationsgerät eingeschaltet ist, wird stets ein Druck zwischen 0,7 bar und 1 bar innerhalb des Druckluftschlauchs 11 aufrechterhalten, unabhängig davon, ob das Ventil 14 geöffnet ist oder nicht. Die automatische Steuerung des Elektromotors 24 mittels des Drucksensors 30 und der Steuereinheit 31 sorgt dafür, daß der Drucklufterzeuger 23 nur dann arbeitet, wenn Aerosol erzeugt wird.

**Patentansprüche**

1. Transportables Inhalationsgerät zur Behandlung erkrankter Atemwege, mit
   - einem Gehäuse (1),
   - einem Drucklufterzeuger (23),
   - einem Elektromotor (24), der den Drucklufterzeuger (23) antreibt,
   - einer Stromversorgung mit wenigstens einem Akkumulator (25),
   - einem vom Gehäuse (1) getrennten Aerosol-Vernebler (7) und
   - einem flexiblen Druckluftschlauch (11), dessen eines Ende mit dem Drucklufterzeuger (23) und dessen anderes Ende an den Aerosol-Vernebler (7) angeschlossen ist,
   **gekennzeichnet** durch

   - ein am Aerosol-Vernebler (7) angeordnetes, handbetätigtes Ventil (14), das die Zufuhr von Druckluft absperrt,
   - eine elektronische Steuereinheit (31) zur Steuerung des Elektromotors (24),
   - einen zwischen Drucklufterzeuger (23) und Ventil (14) angeordneten Sensor (30), der den Druck im Druckluftschlauch (11) überwacht und der elektrisch mit der Steuereinheit (31) verbunden ist, und
   - einen in der Steuereinheit (31) enthaltenen Regler, der bei Druckabfall im Druckluftschlauch 811) den Elektromotor (24) des Drucklufterzeugers (23) anschaltet und bei Erreichen eines oberen Grenzwertes im Druckluftschlauch (11) den Elektromotor (24) abschaltet.

2. Inhalationsgerät nach Anspruch 1, dadurch gekennzeichnet, daß der Regler als Zweipunkt-Regler ausgebildet ist, so daß auch bei Druckabfall im Druckluftschlauch (11) unter einen unteren Grenzwert der Elektromotor (24) des Drucklufterzeugers (23) angeschaltet wird.

3. Inhalationsgerät nach Anspruch 1, dadurch gekennzeichnet, daß der Elektromotor (24) bei einem Druck von unter 0,7 bar angeschaltet wird.

4. Inhalationsgerät nach einem der vorangehenden Ansprüche, dadurch gekennzeichnet, daß der Elektromotor (24) bei einem Druck von mehr als 1 bar abgeschaltet wird.

5. Inhalationsgerät nach einem der vorangehenden Ansprüche, dadurch gekennzeichnet, daß der Elektromotor (24) ein Gleichstrommotor ist.

6. Inhalationsgerät nach einem der vorangehenden Ansprüche, dadurch gekennzeichnet, daß der Drucklufterzeuger (23) ein Membrankompressor ist.

7. Inhalationsgerät nach einem der vorangehenden Ansprüche, dadurch gekennzeichnet, daß der Drucksensor (30) innerhalb des Gehäuses (1) angeordnet ist.

8. Inhalationsgerät nach einem der vorangehenden Ansprüche, dadurch gekennzeichnet, daß der Drucksensor (30) ein Piezo-Element ist.

9. Inhalationsgerät nach einem der vorangehenden Ansprüche, dadurch gekennzeichnet, daß das Ventil (14) am Aerosol-Vernebler (7) durch Druck auf eine Drucktaste (15) betätigt wird.

10. Inhalationsgerät nach Anspruch 9, dadurch gekennzeichnet, daß das Ventil (14) bei gedrückter Drucktaste (15) öffnet, so daß Druckluft vom Druckluftschlauch (11) in den Aerosol-Vernebler (7) einströmt, und beim Loslassen der Drucktaste (15) automatisch wieder schließt.

11. Inhalationsgerät nach einem der vorangehenden Ansprüche, dadurch gekennzeichnet, daß der Druckluftschlauch (11) über eine an der Außenseite des Gehäuses (1) angeordnete Steckbuchse (13) mit dem Drucklufterzeuger (23) verbindbar ist.

12. Inhalationsgerät nach einem der vorangehenden Ansprüche, dadurch gekennzeichnet, daß die Stromversorgung ein Ladegerät (26) für den Akkumulator (25) und ein Netzteil (27) umfaßt.

13. Inhalationsgerät nach einem der vorangehenden Ansprüche, dadurch gekennzeichnet, daß an der Außenseite des Gehäuses (1) ein Einschalter (17) zur Aktivierung der Steuereinheit (31) vorgesehen ist.

**Claims**

1. A transportable inhalation device for the treatment of diseased respiratory ducts comprising
   - a housing (1),
   - an air compressor (23),
   - an electric motor (24) which drives the air compressor (23)
   - a current supply unit comprising at least one accumulator (25)
   - an aerosol atomizer (7) separated from the housing (1) and
   - a flexible compressed air hose (11), one end of which is connected to the air compressor (23) and the other end of which is connected to the aerosol atomizer (7),
   characterised by
   - a manually operated valve (14) which is arranged on the aerosol atomizer (7) and which shuts off the supply of compressed air,
   - an electronic control unit (31) for the control of the electric motor (24),
   - a sensor (30) which is arranged between the air compressor (23) and the valve (14) and which monitors the pressure in the compressed air hose (11) and is electrically connected to the control unit (31) and

   - a regulator which is contained in the control unit (31) and which switches on the electric motor (24) of the air compressor (23) in the event of a fall in pressure in the compressed air hose (11) and switches off the electric motor (24) when an upper limit value is reached in the compressed air hose (11).

2. An inhalation device as claimed in Claim 1, characterised in that the regulator is designed as a two-position regulator so that also when the pressure in the compressed air hose (11) falls below a lower limit value, the electric motor (24) of the air compressor (23) is switched on.

3. An inhalation device as claimed in Claim 1, characterised in that the electric motor (24) is switched on at a pressure of less than 0.7 bar.

4. An inhalation device as claimed in one of the preceding claims, characterised in that the electric motor (24) is switched off at a pressure of more than 1 bar.

5. An inhalation device as claimed in one of the preceding claims, characterised in that the electric motor (24) is a d.c. motor.

6. An inhalation device as claimed in one of the preceding claims, characterised in that the air compressor (23) is a diaphragm-type compressor.

7. An inhalation device as claimed in one of the preceding claims, characterised in that the pressure sensor (30) is arranged inside the housing (1).

8. An inhalation device as claimed in one of the preceding claims, characterised in that the pressure sensor (30) is a piezoelectric element.

9. An inhalation device as claimed in one of the preceding claims, characterised in that the valve (14) on the aerosol atomizer (7) is actuated by the depression of a push-button (15).

10. An inhalation device as claimed in Claim 9, characterised in that when the push-button (15) is depressed the valve (14) opens so that compressed air from the compressed air hose (11) flows into the aerosol atomizer (7) and when the push-button (15) is released the valve (14) automatically closes again.

**11.** An inhalation device as claimed in one of the preceding claims, characterised in that the compressed air hose (11) can be connected to the air compressor (23) via a socket (13) arranged on the exterior of the housing (1).

**12.** An inhalation device as claimed in one of the preceding claims, characterised in that the current supply unit comprises a charging device (26) for the accumulator (25) and a power pack (27).

**13.** An inhalation device as claimed in one of the preceding claims, characterised in that a circuit closer (17) for the activation of the control unit (31) is arranged on the exterior of the housing (1).

**Revendications**

**1.** Inhalateur portatif pour le traitement des maladies des voies respiratoires, comportant:
- un boîtier (1),
- un générateur d'air comprimé (23),
- un moteur électrique (24), qui entraîne le générateur d'air comprimé (23),
- une alimentation électrique, comportant au moins un accumulateur (25),
- un nébulisateur d'aérosol (7) séparé du boîtier (1) et
- un tuyau d'air comprimé (11) flexible, dont une extrémité est raccordée au générateur d'air comprimé (23) et dont l'autre extrémité est raccordée au nébulisateur d'aérosol (7),
caractérisé par
- une soupape (14), manoeuvrée manuellement, disposée sur le nébulisateur d'aérosol (7), qui obture l'amenée d'air comprimé,
- une unité de commande électronique (31), pour assurer la commande du moteur électrique (24),
- un capteur (30), disposé entre le générateur d'air comprimé (23) et la soupape (14), qui surveille la pression dans le tuyau d'air comprimé (11) et est relié électriquement à l'unité de commande (31), et
- un régulateur, sirué dans l'unité de commande (31), qui, en cas de chute de pression dans le tuyau d'air comprimé (11) met en service le moteur électrique (24) du générateur d'air comprimé (23) et, lorsqu'est atteinte une valeur limite supérieure dans le tuyau d'air comprimé (11), met hors service le moteur électrique (24).

**2.** Inhalateur selon la revendication 1, caractérisé en ce que le régulateur est réalisé sous forme de régulateur à deux points, de sorte que le moteur électrique (24) du générateur d'air comprimé (23) soit également mis en service, en cas de chute de pression dans le tuyau d'air comprimé (11) au-dessous d'une valeur limite inférieure.

**3.** Inhalateur selon la revendication 1, caractérisé en ce que le moteur électrique (24) est mis en service lorsque la pression est inférieure à 0,7 bar.

**4.** Inhalateur selon l'une des revendications précédentes, caracrérisé en ce que le moteur électrique (24) est mis hors service lorsque la pression est supérieure à 1 bar.

**5.** Inhalateur selon l'une des revendications précédentes, caractérisé en ce que le moteur électrique (24) est un moteur à courant continu.

**6.** Inhalateur selon l'une des revendications précédentes, caractérisé en ce que le générateur d'air comprimé (23) est un compresseur à membrane.

**7.** Inhalateur selon l'une des revendications précédentes, caractérisé en ce que le capteur de pression (30) est disposé à l'intérieur du boîtier (1).

**8.** Inhalateur selon l'une des revendications précédentes, caractérisé en ce que le capteur de pression (30) est un piézo-elément.

**9.** Inhalateur selon l'une des revendications précédentes, caractérisé en ce que la soupape (14) située sur le nébulisateur d'aérosol (7) est actionnée en pressant sur un bouton-poussoir (15).

**10.** Inhalateur selon la revendication 9, caractérisé en ce que le soupape (14) s'ouvre lorsque le bouton-poussoir (15) est pressé, de sorte que de l'air comprimé provenant du tuyau d'air comprimé (11) s'écoule dans le nébulisateur d'aérosol (7), et se referme automatiquement, lors du relâchement du bouton-poussoir (15).

**11.** Inhalateur selon l'une des revendications précédentes, caractérisé en ce que le tuyau d'air comprimé (11) est susceptible d'être relié au générateur d'air comprimé (23), par l'intermédiaire d'une douille d'enfichage (13) disposée en face extérieure du boîtier (1)

12. Inhalateur selon l'une des revendications précédentes, caractérisé en ce que l'alimentation électrique comprend un chargeur (26) pour l'accumulateur (23) et une partie de réseau (27).

13. Inhalateur selon l'une des revendications précédentes, caractérisé en ce qu'un interrupteur de mise en marche (17) destiné à l'activation de l'unité de commande (31) est prévu en face extérieure du boîtier (1).

# Fig.1

# Fig.2

EP 0 302 958 B1